# EUROPEAN PATENT APPLICATION

(11) **EP 1 356 817 A1**
(43) Date of publication of application: **29.10.2003**
(21) Application number: 01272311.0
(22) Date of filing: 25.12.2001
(51) Int. Cl.: A61K 38/00, A61P 9/10, A61P 27/02, A61P 27/06

(54) **THERAPEUTIC AND/OR PREVENTIVE AGENTS FOR DISEASES DUE TO RETINAL ISCHEMIA**

(30) Priority: 26.12.2000 JP 2000394550
(71) Applicant: ONO PHARMACEUTICAL CO., LTD., Osaka-shi, Osaka 541-8526 (JP)
(72) Inventor: KATSUBE, Nobuo, c/o Ono Pharma. Co. Ltd., Mishima-gun, Osaka 618-8585 (JP); MAEGAWA, Hitoshi, c/o OnoPharma. Co. Ltd., Mishima-gun, Osaka 618-8585 (JP)
(74) Representative: Henkel, Feiler, Hänzel
(86) International application number: JP0111370
(87) International publication number: WO02051431

(57) **Abstract**

An agent for treating and/or preventing for diseases (glaucoma, diabetic retinopathy, macular degeneration, retinal vascular obstruction, and the like) due to retinal ischemia, which comprises, as an effective ingredient, (2R)-N-(1-benzylpiperidin-4-yl)-3-cyclohexylmethylthio-2-((4R)-3-t-butoxycarbonylthiazolidin-4-ylcarbonylamino)propanamide represented by the following formula (I): 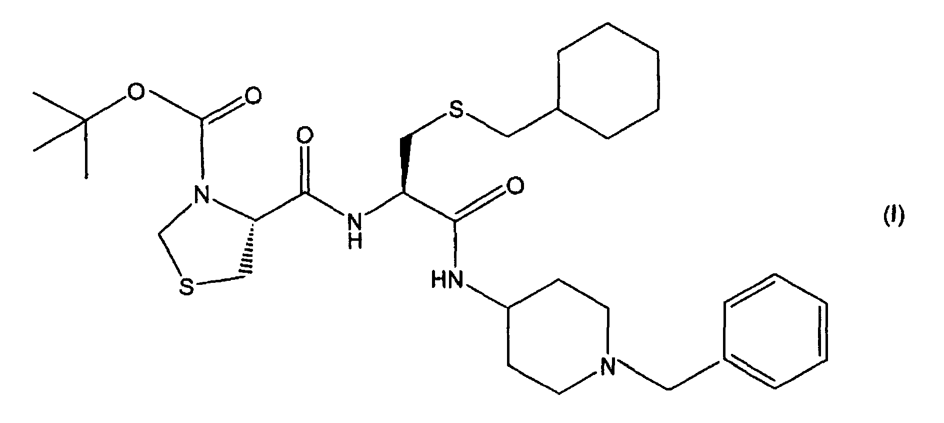 or a nontoxic salt thereof.

## Description

### Technical Field

The present invention relates to an agent for treating and/or preventing diseases due to retinal ischemia.

More specifically, the present invention relates to an agent for treating and/or preventing diseases due to retinal ischemia, which comprises, as an active ingredient, (2R)-N-(1-benzylpiperidin-4-yl)-3-cyclohexylmethylthio-2-((4R)-3-t-butoxycarbonylthiazolidin-4-ylcarbonylamino)propanamide represented by the following formula (I): 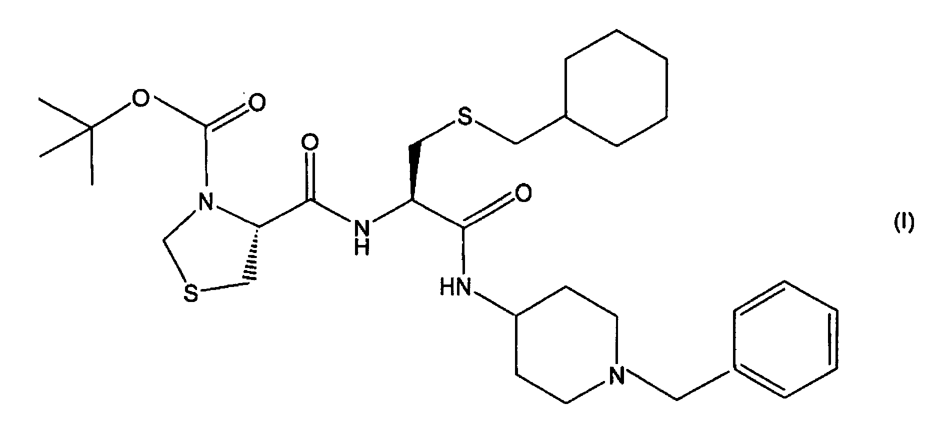 or a non-toxic salt thereof.

### Background Art

The retina is phylogenetically belongs to the diencephalon and is only one central nervous system that can be directly observed from the outside. The retina is a tissue extremely suited for the study of neuronal network, because different from the brain and spinal cord which participate in plural functions, the retina participates in only a single function; visual perception, and its cells for providing visual information to the brain are limited to five types: visual cells,. bipolar cells, ganglion cells, amacrine cells, and horizontal cells. Therefore, a number of neurophysiological studies have been made with the retina as an object. Moreover, the retina occupies an important position in the ophthalmic diseases.

In the past, main ophthalmic diseases were external eye infections such as trachoma. In recent years, however, the proportion of diseases related to visual function has shown an increasing trend. Diseases which cause a deterioration in visual function can be roughly classified into functional diseases such as ametropia (myopia, hyperopia, astigmatism and the like) and accommodation abnormal (presbyopia and the like), and organic diseases such as cataract and glaucoma. Among these diseases, retinal diseases including glaucoma are the most common cause of blindness in the world. In particular, glaucoma is a typical disease which narrows the visual field due to neuronal death of the retina and its main cause has been considered to be a mechanical injury which occur as a result of an increase in intraocular pressure due to impaired outflow of aqueous humor.

In addition to the mechanical damage as described above, glutamate-induced neurocytotoxicity due to retinal ischemia has recently come to be recognized as one of the important causes for retinal disorders in glaucoma. Such a recognition has appeared, because clinical findings which cannot be explained by the theory of mechanical disorder due to an increase in intraocular pressure have been reported, for example, that it is difficult to completely inhibit the progress of a visual field loss due to retinal disorders even if the intraocular pressure is lowered to a normal range; or that as in the case of low tension glaucoma, symptoms of glaucoma appear even when the intraocular pressure falls within the normal range or lower. There is accordingly a demand for the development of not only medical treatment for lowering intraocular pressure but also a medicament directly acting on the retina. However, studies on neuroprotective agents which act on the retina are very rare at present.

On the one hand, a model of retinal disorders due to transient retinal ischemia plays an important role among retinal disorder models. As a result of a study on the effect of the compound represented by the formula (I) using the model, it has exhibited curative effects for retinal disorders by inhibiting retinal neuronal death.

Accordingly, the compound represented by the formula (I) which has curative effects for retinal disorders is considered to be useful as a remedy and/or preventive for many ophthalmic diseases (glaucoma, diabetic retinopathy, macular degeneration, retinal vascular obstruction, etc.) in which involvement of neuronal death due to retinal ischemia has been pointed out.

(2R)-N-(1-Benzylpiperidin-4-yl)-3-cyclohexylmethylthio-2-((4R)-3-t-butoxycarbonylthiazolidin-4-ylcarbonylamino)propanamide represented by the formula (I) is a compound described in Example 2 in the specification of WO00/00470 as an amino acid derivative having an N type calcium channel blocking activity.

According to the description in the specification, the amino acid derivative having an N type calcium channel blocking activity is effective as an agent for preventing and/or treating cerebral infarction, transient cerebral ischemic attack, cerebrospinal disorders after cardiac surgery, spinal blood vessel abnormalities, stress-related hypertension, neuropathy, epilepsy, asthma and pollakiuria, or an analgesic. However, there is no description that the compound represented by the formula (I) or a non-toxic salt thereof is effective for diseases due to retinal ischemia (glaucoma, diabetic retinopathy, macular degeneration, retinal vascular obstruction, etc.).

### Disclosure of the Invention

As a result of intensive investigation, the inventors of the present invention have found for the first time that (2R)-N-(1-benzylpiperidin-4-yl)-3-cyclohexylmethylthio-2-((4R)-3-t-butoxycarbonylthiazolidin-4-ylcarbonylamino)propanamide represented by the formula (I) or a non-toxic salt thereof is effective for diseases due to retinal ischemia and thus completed the present invention.

Thus, the present invention relates to an agent for treating and/or preventing diseases due to retinal ischemia, which comprises, as an effective ingredient, (2R)-N-(1-benzylpiperidin-4-yl)-3-cyclohexylmethylthio-2-((4R)-3-t-butoxycarbonylthiazolidin-4-ylcarbonylamino)propanamide represented by the following formula (I): 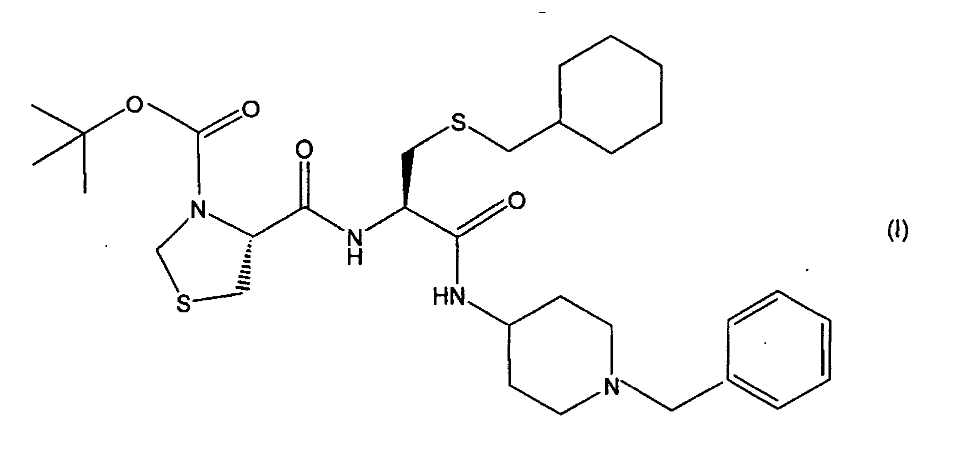 or a non-toxic salt thereof.

The compound represented by the formula (I) may be administered as a salt. As the salt, non-toxic and water-soluble ones are preferred.

Examples of the non-toxic salts include alkali metal salts, alkaline earth metal salts, ammonium salts, amine salts and acid addition salts.

Suitable salts include salts of alkali metals (potassium, sodium, etc.), salts of alkaline earth metals (calcium, magnesium, etc.), ammonium salts, and salts of pharmaceutically acceptable organic amines (tetramethylammonium, triethylamine, methylamine, dimethylamine, cyclopentylamine, benzylamine, phenethylamine, piperidine, monoethanolamine, diethanolamine, tris(hydroxymethyl)aminomethane, lysine, arginine, N-methyl-D-glucamine, etc.).

Suitable acid addition salts, for example, include salts of inorganic acids such as hydrochlorides, hydrobromides, hydroiodides, sulfates, phosphates and nitrates and salts of organic acids such as acetates, lactates, tartrates, benzoates, citrates, methanesulfonates, ethanesulfonates, benzenesulfonates, toluenesulfonates, isethionates, glucuronates, and gluconates.

The compound represented by formula (I) and salts thereof may be converted into the corresponding solvates. As the solvates, non-toxic and water-soluble ones are preferred.

Suitable solvates include solvate salts with a solvent such as water or alcohol (e.g., ethanol, etc.).

### [Preparation process of an effective ingredient]

(2R)-N-(1-Benzylpiperidin-4-yl)-3-cyclohexylmethylthio-2-((4R)-3-t-butoxycarbonylthiazolidin-4-ylcarbonylamino)propanamide represented by the formula (I) or a non-toxic salt thereof can be prepared in accordance with the process as described in the process as described in the specification of WO00/00470.

### [Toxicity]

The compound represented by the formula (I) or a non-toxic salt thereof has markedly low toxicity so that use of it as a pharmaceutical can be considered as safe enough. For example, the minimum lethal dose of the compound represented by the formula (I) by single-dose oral administration to rats was 2000 mg/kg or more.

### Industrial Applicability

### [Application to pharmaceuticals]

As the compound represented by the formula (I) or a non-toxic salt thereof has curative effects on retinal disorders, it is considered to be useful as an agent for treating and/or preventing a number of ophthalmic diseases (glaucoma, diabetic retinopathy, macular degeneration, retinal vascular obstruction, etc.) in which involvement of neuronal death due to retinal ischemia has been pointed out.

When the compound represented by the formula (I) or a non-toxic salt thereof according to the present invention is used for the above-described purpose, it is usually administered systemically or topically via an oral or parenteral route.

Although the dose differs, depending on age, weight, symptom, desired therapeutic effects, administration route, duration of treatment and the like, the compound is usually administered orally at a single dose of 1 mg to 1000 mg per adult once or several times a day; is parenterally administered (preferably, by eye drops) at a single dose of 1 mg to 100 mg per adult once or several times a day; or is intravenously administered continuously for 1 to 24 hours a day.

As described above, the dose varies, depending on various conditions, so that the dose smaller than the above-described dose may be sufficient amount and the dose exceeding the above-described range may be required.

Upon administration, the compound represented by the formula (I) or a non-toxic salt thereof is used as solid compositions, liquid compositions or other compositions, each for oral administration, or as eye drops, eye ointments, injections, external preparations or suppositories, each for parenteral administration.

The solid compositions for oral administration include tablets, pills, capsules, powders, granules and liquids.

The capsules include hard capsules and soft capsules.

Such solid compositions are prepared by mixing one or more active substances with at least one inert diluent such as lactose, mannitol, glucose, hydroxypropyl cellulose, microcrystalline cellulose, starch, polyvinyl pyrrolidone - or magnesium aluminometasilicate. The compositions may contain, in accordance with the conventional process, additives other than the inert diluent, for example, lubricant such as magnesium stearate, disintegrant such as calcium cellulose glycolate, stabilizer such as lactose and solubilizing agent such as glutamic acid or aspartic acid. Tablets or pills may be coated with a film of a gastric soluble or enteric substance such as sucrose, gelatin, hydroxypropyl cellulose or hydroxypropyl methylcellulose phthalate, or with two or more layers, if necessary. Furthermore, capsules made of a substance which can be absorbed in the body, for example, gelatin, is included.

Liquid compositions for oral administration include pharmaceutically acceptable emulsions, solutions, syrups and elixirs. Such liquid compositions contain one or more active substances and an ordinarily employed inert diluent (for example, purified water or ethanol) dissolving the substances therein. These compositions may contain, in addition to the inert diluent, an adjuvant such as a humectant or suspending agent, a sweetening agent, a flavoring agent, an aromatic agent and antiseptic.

The other compositions for oral administration include sprays which contain one or more active substances and are formulated in a manner known *per se* in the art. These compositions may contain, in addition to an inert diluent, a stabilizer such as sodium bisulfite and an isotonic buffer such as sodium chloride, sodium citrate or citric acid. The preparation process of sprays is described in detail in, for example, U.S. Patents Nos. 2,868,691 and 3,095,355.

The dosage form of eye drops for parenteral administration include ophthalmic solutions, ophthalmic suspensions, ophthalmic emulsions and ophthalmic solutions dissolved before use.

Such eye drops are prepared in a known method. For example, an ophthalmic solution is prepared by selecting proper additives from an isotonizing agent (such as sodium chloride or concentrated glycerin), a buffering agent (such as sodium phosphate or sodium acetate), a surfactant (such as "Polysorbate 80" (trade name), polyoxyl 40 stearate or polyoxyethylene hydrogenated castor oil), a stabilizer (such as sodium citrate or edetate sodium) and an antiseptic (such as benzalkonium chloride or p-aminobenzonic acid), if necessary. They are sterilized or subjected to aseptic manipulation in the final step.

For the eye ointments, a known ointment base such as purified lanolin, vaseline, plastibase, liquid paraffin or polyethylene glycol may be used.

Injections for parenteral administration include sterile aqueous and/or non-aqueous solutions, suspensions and emulsions. The aqueous solutions or suspensions include, for example, distilled water for injection and saline. The non-water soluble solutions or suspensions include vegetable oils such as propylene glycol, polyethylene glycol and olive oil, alcohols such as ethanol and "Polysorbate 80" (trade mark). Sterile aqueous and non-aqueous solutions, suspensions and emulsions may be used in combination. Such compositions may additionally contain adjuvants such as antiseptic, humectant, emulsifier, dispersant, stabilizer (such as lactose) and solubilizing agent (such as glutamic acid and aspartic acid). They are sterilized by filtration through a bacteria retaining filter, by the addition of a sterilizer, or by irradiation. They may be prepared in the form of a sterile solid composition such as a freezed-dried product, which may be dissolved in sterile distilled water for injection or another sterile solvent before use.

The other compositions for parenteral administration comprise one or more active ingredient and include liquid preparations for external use; ointments, endermic liniments, suppositories for intrarectal administration and pessaries for intravaginal administration which are formulated in a conventional method.

### Best Mode for Carrying Out the Invention

The present invention will be described specifically by an experimental example. However, that the present invention is not limited thereto.
Effects of the compound represented by the formula (I) (which will hereinafter be called "compound of the present invention") on a rat model with retinal disorders due to transient retinal ischemia;

### [Testing method]

The model with retinal disorders due to transient retinal ischemia was prepared in accordance with the method described in *Eur. J. Pharmaco.,* 350, 53-57 (1998).

After rats (SD male rats, 9-week-old (purchased from Charles River Japan, Inc.)) were weighed, pentobarbital sodium (Pentbarbital Na) (50-75 mg/kg) was intraperitoneally administered to anesthetize them. They were fixed to a platform, and kept warm at around 37°C. A perfusate for ophthalmic surgery ("BSS PLUS"; product of Santen Pharmaceutical) was hung so that the height from the position of the eyes of the rats would be 170 cm ± 5 cm. This height permitted application of pressure of about 130 mmHg. To the perfusate bottle was connected an extension tube and an injection needle (27 gauge x 3/4 inch) was attached to another end of the tube. After pupillary dilatation, the needle was inserted into the anterior chamber of eye from the nose side while the eyelid was opened with forceps and an ischemic burden was applied for 45 minutes (the left eye was treated, while the right eye was left untreated). The compound of the present invention was administered one hour before ischemia was caused. One week after completion of the ischemia treatment, the animals were sacrificed with anesthesia and then, the eyeballs were excised.

After the eyeballs were immobilized in a Davidson's fixative and a paraffin section was prepared at the cross-section passing through the optic papilla, it was dyed with hematoxylin and eosin (H.E.). Photographs of all the cases were taken. The number of granule cells (GCL (ganglion cell layer), cell/mm) per 1 mm width of the optic papilla was counted by optical microscopic examination.

The constitution of the groups and the number of animals are as follows: a compound of the present invention group and a control group, each group consisting of 5 animals, and 10 animals in total.

An aqueous 0.5% carboxymethyl cellulose (0.5% CMC) solution of the compound of the present invention was orally administered at a dose of 100 mg/5 mL/kg, while to the control group was orally administered a 0.5% CMC aqueous solution at a dose of 5 mL/kg.

The results are shown in. Table 1.

**Table 1**

| | The number of granule cells (GCL) (cell/mm) |
|---|---|
| Untreated | 67.4 |
| Control | 40.0 |
| Compound of the present invention | 49.2 |

As is apparent from the above Table 1, the compound of the present invention exhibited a 34% inhibiting activity to the model of retinal disorders due to transient retinal ischemia.

From the results of Table 1, it has been understood that the compound represented by the formula (I) used in the present invention inhibits death of granule cells in the rat model of retinal disorders due to transient retinal ischemia. This suggests that the compound represented by the formula (I) or a non-toxic salt thereof is effective for the treatment and/or prevention of diseases (glaucoma, diabetic retinopathy, macular degeneration, retinal vascular obstruction, etc.) due to retinal ischemia.

## Claims

1. An agent for treating and/or preventing of a disease due to retinal ischemia, which comprises, as an effective ingredient,. (2R)-N-(1-benzylpiperidin-4-yl)-3-cyclohexylmethylthio-2-((4R)-3-t-butoxycarbonylthiazolidin-4-ylcarbonylamino)propanamide represented by the following formula (I): 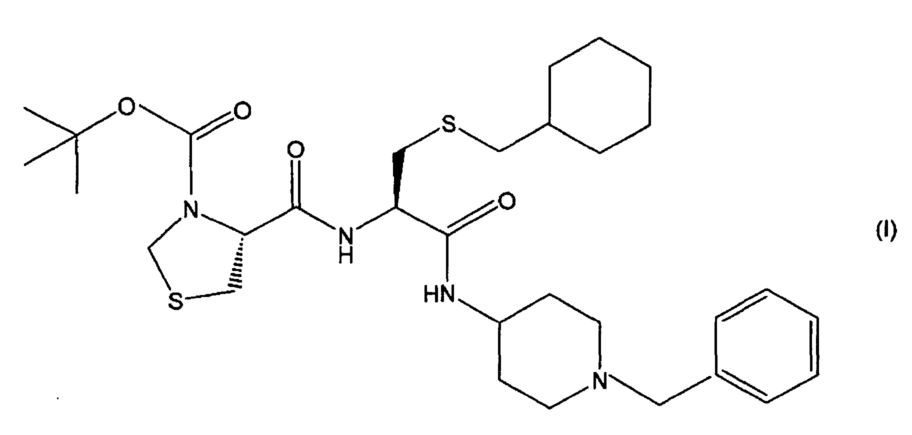 or a nontoxic salt thereof.

2. The agent according to claim 1, wherein the disease due to retinal ischemia is glaucoma, diabetic retinopathy, macular degeneration, or retinal vascular obstruction.
